# EUROPEAN PATENT SPECIFICATION

(11) **EP 0 463 282 B1**
(45) Date of publication and mention of the grant of the patent: **18.10.1995**
(21) Application number: 90830350.6
(22) Date of filing: 27.07.1990
(51) Int. Cl.: A61G 15/14

(54) **A cabin for the protection and de-contamination of the dental armchair unit**
Kabine für die Beschützung und die Dekontaminierung eines zahnärtzlichen Behandlungsstuhls
Cabine pour la protection et la décontamination du fauteuil dentaire

(30) Priority: 18.06.1990 IT 4807490
(43) Date of publication of application: 02.01.1992
(73) Proprietor: De Iorio, Guido, I-00149 Roma (IT)
(72) Inventor: De Iorio, Guido, I-00149 Roma (IT)
(74) Representative: Mascioli, Alessandro, Prof.Dr.

(56) References cited:
- EP-A- 0 278 626
- DE-C- 851 240
- FR-A- 2 133 344
- US-A- 3 439 966
- US-A- 3 537 447
- US-A- 4 252 054

## Description

The present invention concerns a cabin for the protection and de-contamination of the dental armchair unit, for the protection of the personnel and of the patients from the air pollution and from the contamination of surfaces following to the use of instruments spraying (the syringe for spraying air and/or water), of rotating instruments ( a turbine and a micro-motor with cutting point cooled down by compressed and nebulized air and water), of vibrating instruments (tartar ablator with water-cooled point), as well as from the contamination of surfaces produced by operations with "wet fingers" of the dental study personnel.

It is well known that the above mentioned instruments produce in the operative field:
a) water sprays with variable percentages of saliva, blood, single cells, cell aggregates of tissues of the oral cavity, micro-organisms, products of the milling of teeth, food rests;
b) aerosols of little water drops with variable percentages of saliva, blood, single cells, cell aggregates of the tissues of the oral cavity, micro-organisms, products of the milling of the teeth, food rests.

The main functions of the cabin for the protection and the de-contamination are to protect the personnel and the patient from the breathing, from the conjunctival instillation, from the deglutition, from the contact of the teguments with the substances described at points a) and b), of protecting the same above mentioned persons from the superficial contaminations produced by the operations with "wet fingers" by means of contaminated equipment and instruments, of protecting the personnel from the radiations of the x-ray device, of preventing the manipulation and the transport of instruments about the study for performing the conventional washing and sterilization operations, as well as to allow the systematical use of chemical and physical desinfection means that are not useable otherwise.

EP-A-0 278 626 discloses a dental cabin mounted on a vehicle. US-A-3 439 966 discloses an apparatus for providing Patient care in a sterile germ free room.

The aim of the cabin according to the present invention, for the protection and de-contamination, according to the present invention, is the one to put an end to the growing risks in a dental study of getting crossed infections, relating particularly to those contracted due to the figured elements of the blood and of tissue cells (infections caused by the virus of hepatitis B and by the HIV-virus).

For what concerns the details of the structure according to the present invention, it comprises:
- a cabin according to Claim 1.

The present invention will be described more in detail hereinbelow relating to the enclosed drawings, in which a preferred embodiment is shown.
- Figure 1: shows a functional block scheme;
- Figure 2: shows the scheme of the control circuit;
- Figure 3: shows the scheme of the power circuit;
- Figure 4: shows a view of the ceiling and of the perimeter of the cabin according to the present invention;
- Figures 5 and 6: respectively show the walls (1) and (2) provided with a door;
- Figure 7: shows a square section of the cabin according to the present invention;
- Figure 8: shows a complete perspective view.

Relating to the figures, the following details are shown:

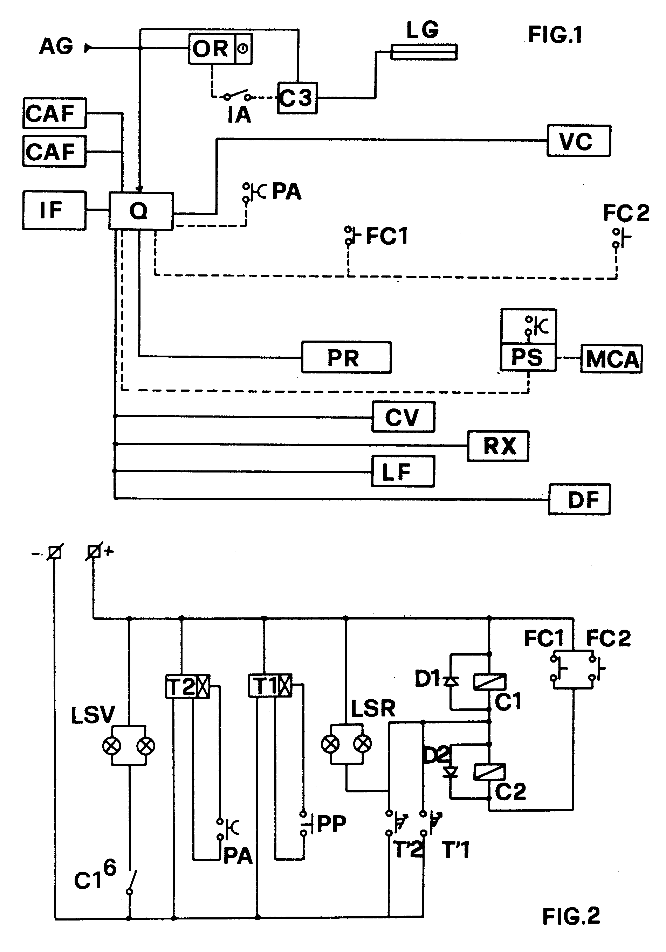

In figure 1, a functional block scheme:
- a feeding net AG from the control board of the dental study;
- a timer OR of the germicidal lamps;
- a manual switch IA that makes OR operate C3;
- a contactor C3 for the germicidal lamps;
- germicidal lamps LG;
- addressed waves interphone IF;
- electric board Q of the de-contamination cabin;
- stop FC1 for door 1;
- stop FC2 for door 2;
- a dental armchair unit PR;
- a pedal unit for the instruments PS;
- power circuits CP;
- auxiliary circuits CA;
- a compressor CAF for a compressed air helmet;
- an electric steam boiler CV;
- a starting T2 switch PA;
- a starting T1 switch PP;
- a switching module MCA for the compressed air;
- a centrifugal fan VE;
- an automatic electric washing machine LF;
- a radiographic device RX;
- a diaphanoscopid lamp DF.

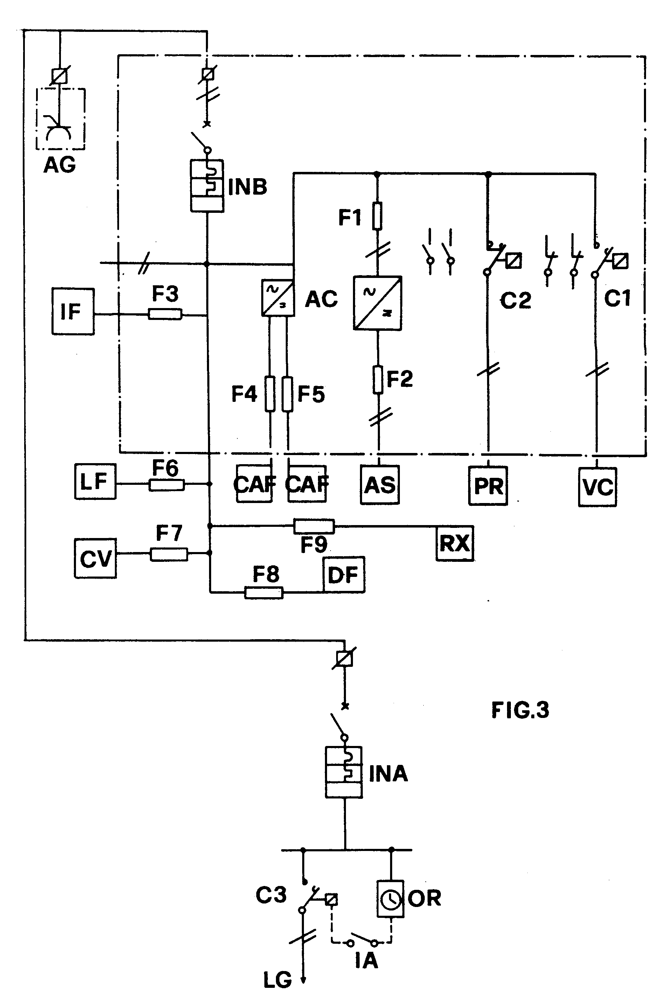

In figure 2, a scheme of a control circuit:
- a green light LSV;
- a sixth contact C1/6 of contactor C1 that lights LSV;
- a timer T2 for an air exchange about each 5 minutes;
- a timer T1 for an air exchange about each 2 minutes;
- a starting T2 switch PA;
- a contact PP of starting T1 pedal;
- a contact n.a. of T2, T2';
- a contact n.a. of T1, T1';
- a red light LSR;
- a contactor C1 for the feeding of a centrifugal fan aspirator VC;
- a contactor C2 for the complexive feeding of the armchair unit;
- diods D1, D2 for the protection on control coils of contactors C1, C2;
- stop FC1 for door 1;
- stop FC2 for door 2;
- a feeding ACC 24 V cc.

In figure 3, a scheme of the power circuit:
- a feeding AG of a net 220 V a.c. of tap CEE 17 2 x 16 A + T;
- a differential switch (2 x 5A, Id = 0,030 A) INA;
- a contactor C3 for the lighting of germicidal lamps;
- a timer OR for the germicidal lamps;
- a manual switch IA that enables OR to operate C3;
- germicidal lamps LG;
- an addressed wave interphonic IF;
- a differential switch INB (4 x 15A, Id = 0,030A) for feeding Q;
- a stabilized feeder AC in continuous current;
- a feeding VC contactor CI;
- a feeding PR contactor C2;
- auxiliaries and signals AS;
- an dental armchair unit PR;
- a centrifugal fan aspirator VC;
- ad addressed waves interphonic IF;
- an automatic electric washing machine LF;
- an electric steam boiler CV;
- fuses F1, F2, F3, F4, F5, F6, F7, F8, F9;
- a diaphanoscopic lamp DF;
- an x-ray device.

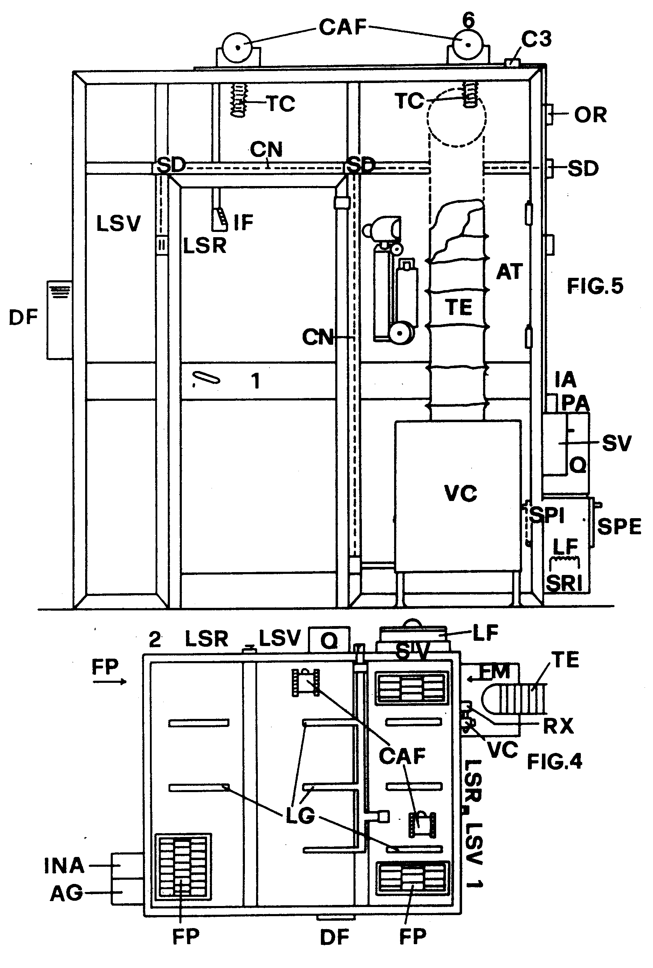

In figure 4, a view of the ceiling and of the perimeter:
- an anti-dust filter FP (air grating);
- an anti-microbic filter FM (air grating);
- a centrifugal fan VC;
- tubes for air output TE;
- feeding AG, net 220 V a.c. on plug CEE 17 2 x 16A + T;
- a differential switch INA (2 x 5A, Id = 0,030A), for the feeding of the germicidal lamps;
- a timer OR for the germicidal lamps;
- a contactor C3 for the lighting of the germicidal lamps;
- germicidal lamps LG;
- an electric board Q of the de-contamination cabin;
- a red light LSR;
- a green light LSV;
- a door 1 = I;
- a door 2 = 2;
- sliding drawers SV;
- an addressed waves interphonic IF;
- a compressor CAF for a compressed air helmet;
- a diaphanoscopic lamp DF;
- an automatic electric washing machine LF;
- an x-ray device RX.

In figure 5, the wall of door 1:
- a manual switch IA that enables OR to operate C3;
- a timer OR for the germicidal lamps;
- a contactor C3 for the lighting of the germicidal lamps;
- an electric board Q of the de-contamination cabin;
- a switch PA for the air exchange each 5 minutes;
- a derivation box SD;
- an inspectionable drain CN;
- a green light LSV;
- a red light LSR;
- stop FC1 for door 1;
- a centrifugal fan aspirator VC;
- a tube TE for the air outlet;
- sliding drawers SV;
- an addressed waves interphonic IF;
- an air compressor CAF for a compressed air helmet;
- a tube TC for feeding air to the helmet CAC;
- a diaphanoscopic lamp DF;
- an x-ray device RX;
- a shutter AT opening inwardly to the cabin;
- an automatic electric washing machine LF;
- an external door SPE of the washing machine LF;
- an internal door SPI of the washing machine LF;
- an internal resistance SRI of the washing machine LF.

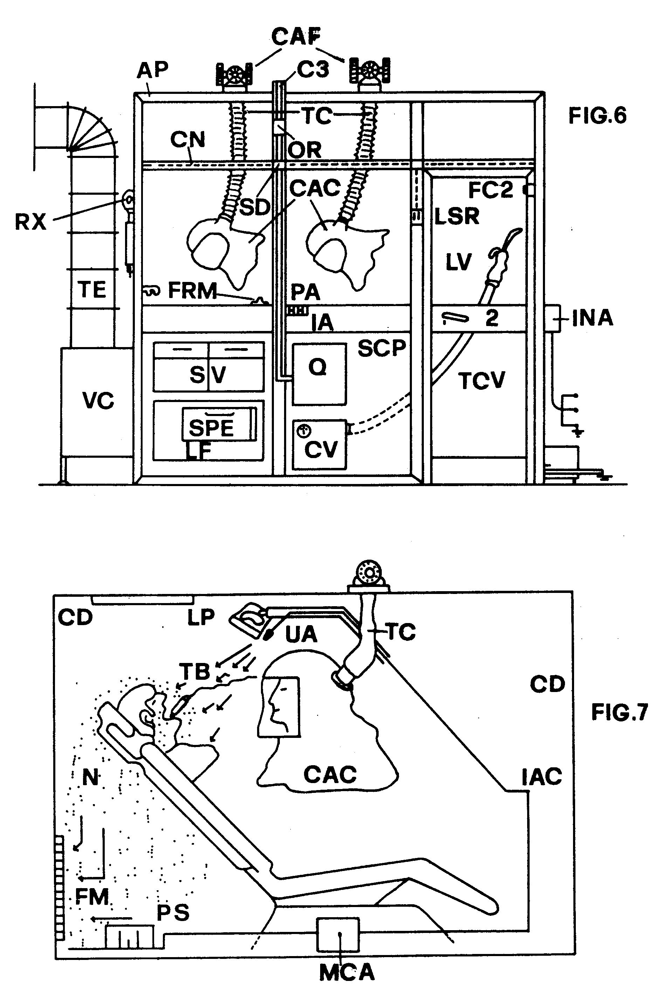

In figure 6, the wall of door 2:
- a differential switch INA (2 x 5A, Id = 0,030A) for the feeding of the germicidal lamps system;
- a manual switch IA that enables OR to operate C3;
- a timer OR for the germicidal lamps;
- a contactor C3 for the lighting of the germicidal lamps;
- an electric board Q of the de-contamination cabin;
- a switch PA for the air exchagne each 5 minutes;
- a red light LSR;
- a green light LSV;
- stop FC2 for door 2;
- a centrifugal fan aspirator VC;
- a tube TE for the air outlet;
- sliding drawings SV;
- shapes AP of pre-painted aluminium;
- an air compressor CAF for a compressed air helmet;
- a tube TC for feeding air to the helmet CAC;
- an inspectionable drain CN;
- a derivation box SD;
- a compressed air helmet CAC;
- stop FRM for shutter AT;
- an x-ray device RX;
- a steam launch LV;
- a tube PCV for carrying the steam;
- an electric steam boiler CV;
- an external door SPE of the washing machine LF;
- an automatic electric washing machine LF;
- a screen SCP for the protection againts x-rays.

In figure 7, a square section of the cabin:
- an air compressor CAF for a compressed air helmet;
- a cabin CD for protection and de-contamination;
- a lamp LP for the dental unit;
- a nozzle UA for the compressed air;
- a tube TC for feeding air to the helmet CAC;
- sprayings and aerosols N;
- an aspiration grating FM with anti-microbic filter;
- pedal PS for the dental instruments unity;
- a module MCA for the air switching;
- a tube IAC for feeding air to nozzle UA.

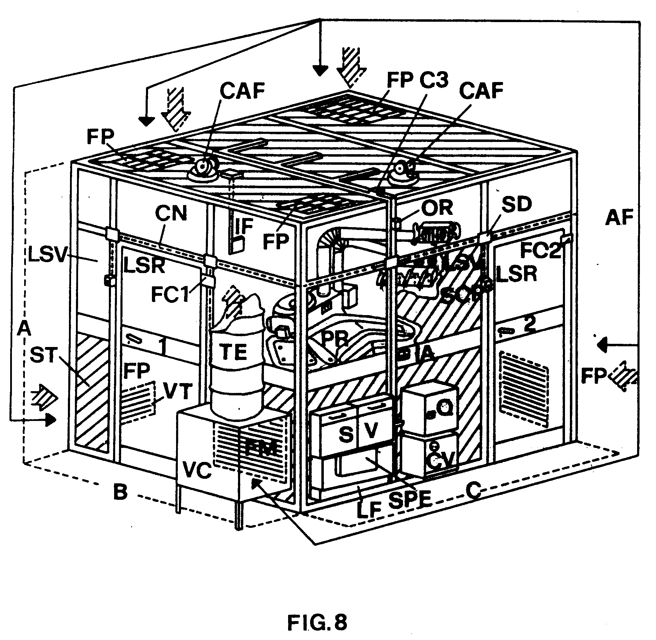

In figure 8, a perspective view:
- height A = 2.30 meters
- width B = 2.00 meters;
- length C = 2.40 meters;
- dental armchair unit PR;
- a manual switch IA that enables OR to operate C3;
- a timer OR for the germicidal lamps;
- a contactor C3 for lighting the germicidal lamps;
- an electric board Q of the cabin;
- a switch PA for the air exchange each 5 minutes;
- derivation boxes SD;
- an inspectionable drain CN;
- a red light LSR;
- a green light LSV;
- stop FC1 for door 1;
- stop FC2 for door 2;
- an air conditioning system AF;
- anti-dust filters FP (air grating);
- anti-microbic filters FM (air grating);
- a centrifugal fan aspirator VC;
- a tube TE for the air outlet;
- an addressed waves interphonic IF;
- a panel of white, bi-shaped bakelite ST;
- an anti-incident glass sheet VT screened againts UV-C;
- an air compressor CAF for a compressed air helmet;
- a door 1 = 1;
- a door 2 = 2;
- sliding drawers SV;
- an automatic electric washing machine LF;
- an outer door SPE of the washing machine LF;
- an electric steam boiler CV;
- an x-ray protection screen SCP;
- an inner door SPI of washing machine LF.

For what concerns the practical realization, the cabin for protection and de-contamination according to the present invention has the following structure:
- a cabin out of white, pre-painted shaped aluminium AP, provided with two entrance doors 1 and 2. The plugging of the frames is performed with panels of white bi-shaped bakelite ST and with sheets of anti-incident glass VT screened against UV-C (see figures 4, 5, 6, 8);
- an electric board Q of fabric glass with a sealing gasket and a key lock; the connections between the board and the peripherical elements are performed by means of copper wire of 0.75 mmq for the control circuit, and with copper wire of 0.25 mmq for the power circuit, passing in inspectionable drains CN with rectangular sections and derivation boxes SD (figures 5, 6, 8);
- a compressed air helmet CAC of the industrial kind, exploiting the air flow coming from air compressor CAF through tube TC with the following advantages: impossibility, for the contamianted air, of getting inside the helmet, due to the over-pressure existing inside the same (PIRELLI SEKURMASTER, SEKURFAN and similar systems), a ventilation that prevents the dimming of the transparent screen and the feeling of heat around the head of the operator, as well as the capacity of providing filtrated air for breathing with a reduction of nearly 100% of the contaminating substances, as the compressor sucks the air from outside the cabin; this kind of protection helmet does not cause resistances on the respiratory organs (figures 4, 5, 6, 7, 8);
- an air nozzle UA with a tube IAC for carrying air and the realtive air switching module MCA that is operated by means of the pedal PS each time an instrument of the dental unit is used. Thus, the nebulized products N are deviated from their trajectory, turned againts the screen of the operator, to the back of the patient and towards the aspiration grating FM, as shown in figure 7;
- a steam boiler CV, electrically fed, that allows to obtain water steam at 5-6 atm for the physical cleaning and disinfection of the resting planes, of the instruments of the eental unit and of all heat-resisting parts inside the cabin; the boiler provides the steam launch LV with steam through tube TCV, as shown in figures 6 and 8;
- an automatic washing machine LF that performs the quick washing of the instruments removing all quantities of contaminating substances and making them, when the washing cycle is completed, absolutely unable of transferring blood, cells and organic fluids. The washing machine is provided with two doors, an internal one SPI and an external one SPE; they allow the loading of the contaminated instruments after the intervention from the inside and the unloading of the same, after the washing and disinfection performed by the washing machine, from the outside, thus preventing to carry the contaminated instruments outside of the cabin around the study. Now said instruments may be placed - without any danger for contamination - into the sterilizer. Said washing machine is provided with an internal resistance RIS with a thermostat and a timer that are able to perform the conventional sterilization cycles at dry heat (200°C x 45-90 minutes), as shown in figures 4, 5, 6, 8;
- an x-ray device RX mounted on a shutter AT opening inwardly to the cabin: when the shutter is opened and blocked by means of stop FRM the radiographic device is addressed towards the patient, and said device is not subject to contamination when it is not used (figures 4, 5, 6);
- a diaphanoscopic lamp DF protected againts the contaminating substances by the glass sheet; the radiograms are fixed on the glass sheet of the cabin (figures 4,5);
- a protection screen SCP againts the radiations produced by the x-ray device RX when working (figures 6, 8);
- a temporized circuit of germicidal lamps LG, shown in figures 1, 3, 4, 5, 6, 8);
- a red light LSR and a green light LSV at doors 1 and 2 (figures 2, 4, 5, 6, 8);
- a forced ventilation system AF, consisting in a centrifugal fan aspirator VC of 1.600 square meters per hour; five immission gratings FP provided with antidust filters, one emission grating FM with an antimicrobic filter, an emission tube TE (figures 4, 5, 6, 7, 8);
- sliding drawers SV for the passage of the instruments to be used and for the outlet of the waste (figures 4, 5, 6, 8);
- an addressed waves interphonic IF with automatic switching of the line, that allows the communication, with free hands, between the cabin and the room in which said cabin is mounted (figures 1, 3, 4, 5, 8).

The cabin for protection and de-contamination according to the present invention may be installed at the following conditions:
a) in the presence of a grounding system according to the rules of the EEC;
b) the room in which the cabin is mounted must have the following minimum dimensions: length 4.00 meters, width 3.80 meters, height 2.80 meters.

The cabin according to the present invention has the following functions:
1) it limits the air contaminations and the surface contaminations that follow the use, in the oral cavity of the patient, of spraying instruments ( air or water syringe), of rotating instruments (turbine and micromotors with the cutting point being cooled by compressed air and water), of vibrating instruments (tartar ablator with water-cooled point), to the sole dental unit PR. Such contamination is caused by little drops of different dimensions, with water base, containing saliva, blood, single and aggregated cells of the oral cavity of the patient, microorganism, products of the milling of the teeth and food rests;
2) it de-contaminates the air during the use of abovementioned instruments by means of the forced air circuit AF that continues its working long after the use of said instruments (for about 120 seconds), until all the air inside the cabin has been changed; at the same time, the red light LSR shows, on doors 1 and 2, 'no entrance', until the green light LSV is lighted;
3) it protects the operator and the assistant by means of the compressed air helmet CAC from the sprayings and the aerosols that arise all around inside the cabin during the interventions, as well as the operator and the assistant from the chemical and physical disinfectants used for the de-contamination (cleaning with acid glutaraldeid followed by a steam jet);
4) it exploits the effect of aerodynamic barrier produced by the air jet that comes from nozzle UA from under the lamp of the dental unit; this allows to avoid, when the operator comes frontally or laterally near to the patient, sprayings and aerosols directed againts the transparent screen of the compressed air helmet CAC which, after a few seconds of use of one of the instruments of the unit (turbine TB, micromotor etc.), would reduce the visibility of the operator. By means of thus device, the greatest part of the sprayings and of the aerosols are pushed to the back of the patient towards the aspiration grating FM.
5) It de-contaminates the air and the surfaces after every intervention: the assistant will carefully take care of permorming the cleaning of the internal surfaces contaminated during the intervention with an efficacious disinfectant like acid glutaraldeide followed, only on the heat-resisting surfaces, a jet of steam, by means of the launch LV, at 5-6 atm, coming from the boiler CV. During this phase, the assistant takes care of his own protection by wearing a feetlong overall together with lactice gloves and a compressed air helmet identical to the one of the operator; then, when the assistant has left the cabin, after having closed door 1, by pushing switch PA, he starts a long air exchange (5 minutes); thus the internal air is changed oftenly, allowing to eliminate the fumes of the disinfectants and to dry the surfaces from the rests of steam and condensation;
6) it allows the passage from the inside to the outside and vice versa, by means of the sliding drawers SV, of the instruments and of the one-use material that has been contaminated and that is to be eliminated without thereby contaminating the room in which the cabin has been mounted and in which the remaining part of the devices , instruments and materials are kept;
7) it performes, by means of the washing machine LF, of the instruments used during the interventions, with a quick cycle that takes about three minutes: thus the manipolations of the contaminated instruments are reduced to a minumum, avoiding to carry them about in the dental study. The operation of loading the washing machine is performed, at the end of each intervention, by the operator directly through the door SPI that opens inside the cabin. Once the instruments have been washed, they may be sterilized, being free of blood, cells and organic fluids, particularly dangerous for the transmission of the HIV and the HBV. The washing machine performs washing with 90°C water using the apposite disinfecting cleaning substance. The steam that comes out when the door of the washing machine is opened is taken away by aspiration grating FM with anti-microbic filter. Through the external door SPE the instruments may be led to sterilization, which does not form the object of the present invention, making use of the gratings for carrying the instruments provided in said washing machine. On the contrary, the sterilizing function of the washing machine may be used, being provided with a resistance RIS able to determine temperatures up to 200°C, as well as of a timer and a thermostat that control the time periods and the use temperatures;
8) it avoinds the usual contamination with contamination substances from the oral cavity of the patient of the x-ray device RX, that is brough inside the cabin bringing the shutter AT inside and fixing it with the apposite stop FRM;
9) it avoids the contamination of the diaphanoscopic lamp DF fixed on the external side of the cabin, behind one of the plugging glass sheets, and the radio-grams are fixed, for the reading thereof, directly on the surface of the glass sheet, on the internal side of the cabin;
10) it protects the personnel, through the screened wall SCP, againts the radiations of the x-ray device RX when it is working: the shooting of the radiogram may be taken with the operator outside the cabin, being therefore protected by the screened wall, and the patient sitting down on the dental armchair unit and the x-ray device RX put in use position with the shutter AT inwardly opened;
11) it performs the disinfection cycle of the internal surfaces and of the dental unit PR by means of germicidal lamps, the lighting thereof being controlled, in hours when there is nobody in the dental study (at night), by a 24 hour timer and by a switch consent that allows to manually stop, or not, the lighting of the lamps. The same kind of disinfection is efficacious on the inner surfaces of the cabin and of the floor of said cabin; the glasses provided with screening againts UV-C, and all remaining structural and accessory parts of the cabin prevent, however, the irradiation outside said cabin.

The cabin for protection and de-contamination according to the present invention is therefore used in the the anti-microbic filter of aspiration grating FM; should in this time period the accidental opening of doors 1 and 2 occur the stops FC1 and FC2 would lead a positive control tension to the coil of the contactor C2; in that moment, said coil receives, through T2', the negative pole of the control tension: thus the contact n.c. of C2 would be opened and the feeding tension would be taken off from the unit PR; thus a wrong operation is signalled (figures 1, 2, 3) before introducing a further patient.

At the moment of use, the patient gets in and sits down on the dental armchair unit PR passing through door 2; he is protected by an antistatic clothing provided with shoes and gloves. Also the operator enters inside the cabin through door 2 which he closes behind him, wearing immediately the protection helmet CAC, provided with a feeding of filtered and compressed air taken from outside said cabin.

The intervention may start: the operator asks the assistant, who is outside the cabin, for the instruments needed and they are introduced through the sliding drawers (function 6, figures 4, 5, 6, 8).

Now the use of any of the instruments of the dental unit starts, making use of any of the functions 1, 2, 3, 4, 8, 9; the contamination is limited to the dental unit PR which, during the use of contaminated instruments, has a negative pressure gradient with respect to the room, caused by the forced ventilation AF that, together with the sealing of said cabin, prevents the propagation of the contamination to the outside; the centrifugal fan VC of the circuit AF is operated each time one of said instruments is used, and on the pedal of the dentalunit PR the contact of the switch PP is closed and through the air switching module MCA, the air jet that gets out of nozzle UA - below lamp LP - is controlled, and thus function 4 is operated. The protection of the screen and of the helmet CAC is obtained againts the sprayings and the aerosols, that otherwise would dirten them reducing the visibility. These contaminating substances are led, just when they come from the oral cavity of the patient, to the his back towards grating FM; the operator controls timer T1 which, by means of the contact T1', brings the negative of the feeding to the coils of the contactors C1 and C2; by means of a contact n.a., C1 feeds the centrifugal fan VC, and C2 takes the feeding to the dental unit PR by means of a contact n.c., when the accidental opening of doors 1 and 2, through the stops FC1 adn FC2, controls its coil. So the contaminating action of the instruments of the dental unit PR is interrupted; the timer T1 has the feature of closing the contact T1' at the same time the contact PP is closed, and keeps it closed for further 120 seconds after the release of the contact PP. Thus, to each use of the contaminated instruments follows the automatic complete air exchange. The contactor C2 needs, for switching, the contemporary control of the stops FC1 and FC2 and of one of the contacts T1', T2' responsible for the starting of VC (figures 1, 2, 3, 4, 5, 6, 7, 8). When the complete air exchange has been completed, the red light LSR on the doors gets off and the green light LSV is lit 'free entrance' (figures 2, 4, 5, 6, 8). When the intervention is completed, the contaminated instruments are led out of the cabin, according to functions 7 and 8, introducing the same into washing machine LF through the inner door SPI, and the washing cycle is started (three minures with 90°C water) with chemical cleaning and disinfecting substances. When the cycle is completed the instruments may be taken from outside the cabin, through external door SPE, without any organic fluid, cells or blood. If no more interventions are to be performed, it is possible to use the sterilizing function of the washing machine, starting for the appropriate time periods (45-90 minutes) the resistance SRI, that brings the internal temperature up to 200°C. The wastes are collected in an apposite closed bag and led outwardly for the following elimination through the outlet sliding drawer. On doors 1 adn 2 the green lights LSV are lit: the air has been de-contaminated and therefore the patient and the operator may come out. If during the intervention the help of the assistant is needed, he may go in and out through door 1 only when the green light LSV is lit; as above described, C2 is the contactor that intervenes in caso of accidental entrance with the red light lit. Now the cabin is free again (patient and operator have left): functions 5 and 7 may start again for preparing the installation for receiving a new patient who, when entering the cabin, will find the air completely de-contaminated and the surfaces washed and disinfected in an appropriate manner, without disturbing fumes (figures 1, 2, 3, 4, 5, 6, 7, 8).

For using the x-ray devcie RX, function 8 is used: opening shutter AT to which the x-ray device is fixed, and blocking said shutter with stop FRM, it is possible to bring said device near to the head of the patient and to use it for performing the necessary exams. By means of this operation, it is avoided to expose the x-ray device RX to contamination when it is not used. The device is therefore introduced by the shutter inside the cabin only for that time period necessary for its use. During the emission of ionogenic x-radiations, the personnel finds protection behind the screened wall SCP, function 10. The diaphanoscopic lamp DF mounted behind a glass sheet of the cabin is free from the mentioned contaminations (function 9).

## Claims

1. A cabin for the protection and de-contamination of a dental armchair unit, for limiting the contamination of the surfaces of the devices and of the inner parts of the cabin, contamination coming from the oral cavity of the patients and that gets dispersed following to the use of the instruments and to the operations that during and after the interventions cause air and surface contamination, with an air conditioning system that inside the cabin, closed on all six walls, exploits the presence of the internal depression, making the outlet of the contamination through little structural flaws, through gratings (FP), through sliding drawers, while opening their cap, impossible, and allowing the exchange of the contaminated air with pure air, so as to prevent that the contaminating substances get freely dispersed in the external ambient, and this all so as to prevent the mechanism of the cross-infections in a dental ambient, obtained by means of the protection of the personel and of the patient and by means of the de-contamination of the surfaces and of the air, further comprising:
- a steam boiler (CV) used as a physical cleaning and disinfection means;
- a diaphanoscopic lamp (DF) in a position protected, behind a glass sheet, from the contaminating substances;
- a complex of a shutter (AT), opening inwardly to the cabin, and of a radiographic device (RX), positioned externally to the cabin, that is protected, when not in use, against contaminating substances by the shutter closed;
- a washing machine (LF) for the instruments, that prevents the outlet out of the cabin of the contamined instruments;
- a protection screen (SCP) against ionogenic x-radiations, out of one piece with the cabin, outwardly keeping the protection against the contaminating substances;
- a compressed air protection helmet (CAC) that, in the particular mounting of the cabin, sticks the air directly from a clean ambient, i.e. outside said cabin, increasing to 100% its capacity of reducing the contaminating substances with respect of the sole filter functions of the compressor (CAF) provided in a contaminated ambient;
- a germicidal lamps system (LG) applied by exploiting the structural features of the cabin.

2. A protection and de-contamination cabin according to claim 1, characterized in that a compressed air jet comes out of a nozzle provided below said germicidal lamps (LG).

3. A cabin for the protecting and de-contamination, according to claim 1, characterized in the presence of an electric steam boiler (CV) with a steam launch (LV) and tubes for carrying the steam (PCV) which forms, together with the cabin and its air conditioning system (AF), a single functional unity that allows to use the steam as a physical cleaning and disinfection means.

4. A protection and de-contamination cabin according to claim 1, characterized in that said germicidal lamps (LG) provide - being placed inside said cabin - for the physical disinfection in the times of non-use, without the danger for persones outside said cabin, of getting accidentally exposed to the ultraviolet radiations.

5. A cabin for the protection and de-contamination, according to claim 1, characterized in the washing machine (LF) having a double internal (SPI) and external (SPE) door, having also sterilization functions, and in the cabin in a single functionality unit that prevents the outlet from said cabin of the instruments contaminated during the interventions, having the purpose, in synergy with all other parts of the cabin according to the present invention, to prevent the catching of cross-infections in the dental study.

6. A protection and de-contamination cabin according to claim 1, characterized in that said diaphanoscopic lamp (DF) is protected against the contaminating substances by a glass sheet.

7. A cabin for the protection and de-contamination, according to claim 1, characterized in that it comprises a wall screened against ionogenic x-rays (SCP), for the protection, while using the x-ray device, of the personnel.

## Patentansprüche

1. Schutz-und Dekontaminationskabine für einen Zahnarztstuhl mit Zubehör, zur Einschränkung der Kontamination der Oberflächen der Vorrichtung und des Inneren der Kabine, wobei die Kontamination der Mundhöhle der Patienten entspringt, und sich im Raum infolge des Gebrauches der Instrumente und der Eingriffe während und nach den Operationen verbreitet und dabei zur Luft-und Oberflächenkontamination führt, mit einem Pressluftsystem, welches innerhalb der Kabine, die auf allen sechs Seiten geschlossen ist, die interne Depression ausnutzt, und es dadurch unmöglich macht, daß sich die Kontamination auf Grund kleiner Strukturfehler durch Gitter (FP), durch Gleitvorrichtungen während der Offnung der Abdeckvorrichtung verbreitet und das Auswechseln der kontaminierten Luft mit reiner Luft gestattet, und dadurch verhindert, daß sich die kontaminierenden Elemente frei außerhalb der Kabine verbreiten, und dadurch den Mechanismus der gekreuzten Infektionen im zahnärztlichen Milieu unterbinden, mit Mitteln zum Schutz des Personals und des Patienten und Mitteln zur Dekontamination der Oberflächen und der Luft, bestehend aus:
- einem Dampfkessel (CV), verwendet als physikalisches Reinigungs-und Desinfektionsmittel;
- einer diaphanoscopischen Lampe (DF) in geschützter Stellung in Bezug auf die kontaminierenden Substanzen, hinter einer Glasplatte;
- einer Abdeckvorrichtung (AT), die zur Innenseite der Kabine hin geöffnet werden kann, und einer Röntgenvorrichtung (RX), die sich außerhalb der Kabine befindet, und die während der Zeit des Nichtgebrauches gegen die kontaminerenden Substanzen durch die geschlossene Abdeckvorrichtung geschützt ist;
- einer Waschmaschine (LF) für die Instrumente, die das Austreten der kontaminierten instrumente aus der Kabine verhindert;
- einem Schutzschirm (SCP) gegen ionisierende Strahlen (X), der fest mit der Kabine verbunden ist, der den Schutz gegen die kontaminierten Substanzen außerhalb der Kabine gewährleistet;
- einem Schutzhelm (CAC) mit Pressluft, der Dank der besonderen Montage der Kabine die Luft direkt aus dem reinen Milieu entnimmt, d.h.von außerhalb der Kabine, und dadurch die Vermeidung der Kontaminationsgefahr um 100% erhöht, verglichen mit nur der Wirkung der Filter des Kompressors (CAF), der sich in einem kontaminierten Milieu befindet;
- einer Vorrichtung keimtötender Lampen (LG), die unter Ausnutzung der strukturellen Beschaffenheit der Kabine angebracht wird.

2. Schutz-und Dekontaminationskabine nach Anspruch 1, dadurch gekennzeichnet, daß ein komprimierter Luftstrahl aus einer Offnung austritt, die unter den keimtötenden Lampen (LG) angebracht ist.

3. Schutz-und Dekontaminationskabine nach Anspruch 1, durch einen Dampfkessel mit entsprechendem Dampfausdrücker und Dampfrohr gekennzeichnet, die mit der Kabine und ihrer Luftdruckanlage eine Funktionseinheit bilden, die es ermöglicht, den Dampf als physisches Mittel zur Reinigung und desinfektion zu verwenden.

4. Schutz-und Dekontaminationskabine nach Anspruch 1, dadurch gekennzeichnet, daß besagte keimtötende Lampen (LG), die sich innerhalb der Kabine befinden, die physische Desinfektion während der Zeit des Nichtgebrauches vornehmen, ohne Gefahr der zufälligen Aussetzung von Ultraviolettstrahlen der Personen, die sich außerhalb der Kabine befinden.

5. Schutz-und Dekontaminationskabine nach Anspruch 1, gekennzeichnet durch die Schnellwaschmaschine mit doppelter Inner-Außenklappe, die auch die Funktion eines Sterilisators hat, und durch die Kabine, die eine einzige Funktionseinheit bilden, die das Austreten aus der Kabine der kontaminierten Instrumente während der Operationen verhindert, mit dem Zweck, im Einklang mit allen anderen Elementen der Erfindung zu verhindern, daß sich Querinfektionen innerhalb der Zahnarztpraxis bilden.

6. Schutz-und Dekontaminationskabine nach Anspruch 1, dadurch gekennzeichnet, daß die diaphanoscopische Lampe (DF) gegen die kontaminierenden Materialien durch eine Glasscheibe geschützt ist.

7. Schutz-und Dekontaminationskabine nach Anspruch 1, dadurch gekennzeichnet, daß sie mit einem Schutzschirm gegen ionisierende Strahlen (SCP), zum Schutz des personals während des Gebrauches der Röntgenanlage, versehen ist.

## Revendications

1. Cabine de protection et décontamination pour un ensemble fauteuil-appareillage odontologique, du type apte à circonscrire, relativement aux surfaces des appareillages et aux parois intérieurs de la cabine, les matériaux de contamination que viennent de la cavité orale des patients, que se dispersent à la suite de l'usage des instruments et à la suite des maneuvres qui provoquent, pendant et aprés les interventions, la contamination de l'air et de la surface, avec l'installation d'air forcée, la quelle, dans la cabine fermée aux six côtés, utilise la présence de la dépression à l'intérieur, de façon à rendre impossible la sortie des matériaux de contamination à cause de petits défauts structurels, à travers des grilles (FP), à travers des tiroirs à glissiére pendant l'ouverture du couvercle, et permit le rechange de l'air contaminée avec de l'air saine, de façon à éviter que les matériaux de contamination se repandent librement dans le millieu extérieur, pour obténir l'interdiction des mécanismes d'infection croisées dans le millieu odontologique, obténue avec moyens de protéction du personnel et du patient et avec moyens de décontamination des surfaces et de l'air, y compris:
- une chaudière du vapeur (CV), utilisée comme moyen de détersion-désinfection fisique;
- une lampe diaphanoscopique (DF), dans une position telle a être protégée, derrièr un panneau de vitre, des matériaux de contamination;
- un ensemble-volet (AT), ouvrable en direction de l'intérieur de la cabine, et radiographique (RX) à l'exterieur de la cabine lequel, pendant le périod de non-usage, est protégé contre les matériaux de contamination par le volet fermé;
- une machine à laver pour les instruments, qu'empêche la sortie des instruments contaminés à l'extérieur de la cabine;
- un écran de protection (SCP) contre les radiations ionisants (X), d'une seule pièce avec la cabine, en conservant la protection vers les matériaux de contamination à l'extérieur;
- un casque de protection à l'air forcée (CAC), lequel, pendant le montage de la cabine, prend l'air directement dans le millieu sain, c'est à dire à l'extérieur de la cabine, de façon à augmenter du 100% la capacité d'atténuer la contamination en comparaison du cas de l'action des seules filtres du compresseur (CAF), que se trouve dans un millieu contaminé;
- un installation à lampe germicide (LG), qu'on applique utilisant les caractéristiques structurels de la cabine.

2. Cabine de protection et décontamination selon la revendication 1, caractérisée en ce que un jet d'air comprimé sort par une buse, appliquée sous les lampes germicides (LG).

3. Cabine de protection et décontamination selon la revendication 1, caractérisée par la présence d'une chaudiére du vapeur avec lance rélative et tubage de porté du vapeur que forme, avec la cabine et son installation d'air forcée, une seule unité fonctionelle, apte à permettre l'usage du vapeur comme moyen fisique de détersion et désinfection.

4. Cabine de protection et décontamination selon la revendication 1, caractérisée en ce que dites lampes germicides (LG), que se trouvent à l'intérieur de la cabine, pourvoient à la désinfection fisique, pendant le temps de non-usage, sans péril d'exposition accidentelle aux radiations ultra-violets pour les personnes que se trouvent à l'extérieur de dite cabine.

5. Cabine de protection et décontamination selon la revendication 1, caractérisée par la machine à laver rapide à portière intérieure-extérieure, avec fonction aussi de stérilisateur, et de la cabine, que forment un bloc unique fonctionel, qu'empêche la sortie des instruments contaminés de la cabine pendant les opérations, avec le but, en synergie avec toutes les autres éléments restants de l'invention, d'empêcher que se produisent des infections croisées dans le cabinet odontologique.

6. Cabine de protection et décontamination selon la revendication 1, caractérisée en ce que la lampe diaphanoscopique (DF) est protégée contre les matériaux contaminants par le panneau de vitre.

7. Cabine de protection et décontamination selon la revendication 1, caractérisée par le fait qu'elle comprend un paroi de protection au moyen d'un écran contre les radiations ionisants (SCP) pour la protection, pendant l'usage de l'appareillage radigraphique, du personnel.
